# EUROPÄISCHE PATENTSCHRIFT

(11) **EP 3 708 018 B1**
(45) Veröffentlichungstag und Bekanntmachung des Hinweises auf die Patenterteilung: **10.03.2021**
(21) Anmeldenummer: 19161972.5
(22) Anmeldetag: 11.03.2019
(51) Int. Cl.: A43B 7/14, A43B 17/00

(54) **SCHUHEINLEGESOHLEN-ROHLING UND VERFAHREN ZUM INDIVIDUELLEN ANPASSEN EINES SOLCHEN SCHUHEINLEGESOHLEN-ROHLINGS**
SHOE INLAY-SOLE BLANK AND METHOD FOR CUSTOMISING SAME
SEMELLE INTÉRIEURE DE CHAUSSURE ET PROCÉDÉ D'AJUSTEMENT INDIVIDUELLE D'UNE TELLE SEMELLE INTÉRIEURE DE CHAUSSURE

(43) Veröffentlichungstag der Anmeldung: 16.09.2020
(73) Patentinhaber: Spannrit GmbH, 63801 Kleinostheim (DE)
(72) Erfinder: Katzer, Roland, 63801 Kleinostheim (DE)
(74) Vertreter: Erhardt, Martin

(56) Entgegenhaltungen:
- WO-A1-2007/086806
- DE-A1-102009 035 354
- DE-U1- 20 023 245

## Beschreibung

Die Erfindung betrifft einen Schuheinlegesohlen-Rohling, speziell einen Schuheinlegesohlen-Rohling, welcher in der Orthopädie für die individuelle Anpassung an die Fußsohle eines Trägers der Schuheinlegesohle verwendbar ist.

Solche Schuheinlegesohlen-Rohlinge können aus verschiedenartigsten Materialen hergestellt werden, wobei in neuerer Zeit die Verwendung von PU-Schaumkunststoff weit verbreitet ist. Dabei können diese Rohlinge aus einem PU-Schaumkunststoff-Plattenmaterial durch Materialabtrag mittels Fräsen und/oder Schleifen hergestellt werden oder bevorzugt mittels RIM-Verfahren, wobei hier der Schaumkunststoff innerhalb einer Schäumform durch das Zusammenbringen eines Polyols mit einem Isocyanat innerhalb der geschlossenen Schäumform entsteht. Dabei kann auch ein sog. Fräsblock erzeugt werden, der zum Einbringen eines orthopädisch wirksamen Fußbettes auf der Oberseite des Rohlings mittels Fräsen oder Schleifen bearbeitbar ist. In einer anderen Ausführungsform entsteht in der Ausschäumform ein Schuheinlegesohlen-Rohling mit einem direkt durch die Schäumform ausgebildeten orthopädisch wirksamen Fußbett. Derartige in der Orthopädie verwendeten Schuheinlegesohlen-Rohlinge weisen dabei üblicherweise ein vorgeformtes orthopädisch wirksames Fußbett auf, welches insbesondere durch Orthopäden und/oder Orthopädietechniker individuell an die Fußform und/oder Schuhinnenform des Trägers der Schuheinlage angepasst wird. Dabei gibt es je nach zu behandelnder Fußfehlstellung bzw. Fußform verschiedene Grundformen, von denen ausgehend der Orthopäde bzw. der Orthopädietechniker die Sohle individuell anpassen kann.

Zur Erhöhung des Tragekomforts als auch zum Erreichen weiterer gewünschter orthopädischer und nicht-orthopädischer Eigenschaften der Schuheinlegesohle, beispielsweise einer Rutschfestigkeit innerhalb des Schuhs, werden die Schuheinlegesohlen-Rohlinge auf der Oberseite und/oder Unterseite mit geeigneten Decken versehen. Diese Decken können dabei ganzflächig oder auch nur teilweise, einlagig oder mehrlagig auf den Grundkörper des Schuheinlegesohlen-Rohlings aufgebracht sein.

Aus EP 1 967 086 B1 ist ein im RIM-Verfahren erzeugbarer Fräsblock bekannt, bei welchem durch Fräsen und/oder Schleifen auf der Oberseite ein orthopädisch wirksames Fußbett eingebracht werden kann. Weiter ist in EP 2 929 792 B1 eine im RIM-Verfahren herstellbarer orthopädischer Schuheinlegesohlen-Rohling mit einer im Wesentlichen schuheinlegesohlen-artigen Grundform gezeigt, der auf einer Oberseite ein aus dem Schuhkunststoff dreidimensional geformtes Fußbett zeigt. Bei letzterem Verfahren wird das dreidimensional geformte Fußbett durch die geeignete Ausbildung der Ausschäumform direkt während des RIM-Schäumvorganges ausgebildet.

DE 10 2009 035 354 A1 zeigt eine orthopädische Einlage für einen Sicherheitsschuh mit einem an den Fuß des Trägers individuell angepassten Kern, auf dessen dem Fuß zugewandten Seite mittels eines Klebers ein textiler Bezug aufgeklebt ist. Eine solche orthopädische Einlage, die kostengünstig herzustellen ist und dennoch die anti-statischen Erfordernisse von Sicherheitsschuhen erfüllt, wird dadurch erreicht, dass der Kern aus einem einzigen Material gebildet ist, dem Grafit beigemischt ist.

DE 200 23 245 U1 zeigt eine individuell anpassbare, insbesondere orthopädische Schuheinlage, welche Schuheinlagenelemente in Form eines anatonisch geformten Rohlings, zumindest einer Pelotte und zumindest eines Deckenelements aufweist. Der Rohling ist zumindest auf seiner zur Fußsohle hin gerichteten Oberfläche mit dem zumindest diese komplett abdeckenden Deckelement, einer Oberdecke, abgedeckt. Die Pelotte ist zwischen dem Rohling und dem Deckelement angeordnet. Zumindest weist der Rohling und/oder das Deckelement auf ihrer einander zugewandten Oberfläche ein thermisch wiedererweichbares Klebemittel auf, durch welches die Schuheinlagenelemente mehrfach lösbar und wiederbefestigbar miteinander verbunden sind.

Bei den aus zuvor genannten Stand der Technik bekannten Schuheinlegesohlen-Rohlingen können sowohl auf der Oberseite als auch auf der Unterseite Decken aufgebracht werden. Hier sollte jedoch das Aufbringen einer solchen Decke erst dann erfolgen, wenn die Schuheinlegesohle der individuellen Fußform des Trägers der Schuheinlegesohle angepasst ist, also der Grundkörper fertig bearbeitet ist. Der Stand der Technik, insbesondere EP 2 929 792 B1, schlägt hierzu vor, die Decke vor dem Aufbringen auf das dreidimensional geformte Fußbett - also nach dem Anpassen an die Fußform des Fußbettes - auf der Rückseite mit einer TPU-Schicht zu versehen, mittels welcher die Decke ohne weitere Hilfs- oder Klebstoffe auf den Schaumkunststoff aufgebügelt werden kann. Diese TPU-Schicht wird dabei während des Bügelvorgangs gleichzeitig mit dem Schuheinlegesohlen-Rohling soweit erhitzt, dass sowohl das Material des Schuheinlegesohlen-Rohlings als auch die TPU-Schicht über deren jeweilige Schmelztemperatur erwärmt werden und so miteinander verschmelzen bzw. verschweißen können. Somit ist die einmal aufgebrachte Decke nicht wieder zerstörungsfrei vom Schuheinlegesohlen-Grundkörper ablösbar. Eine weitere Anpassung des Schuheinlegesohlen-Körpers auf derjenigen Ober- oder Unterseite, auf den die Decke aufgebracht ist, ist somit nicht mehr ohne Weiteres möglich.

Es ist daher Aufgabe der Erfindung einen orthopädisch wirksamen Schuheinlegesohlen-Rohling bereitzustellen, welcher ein dreidimensional ausgebildetes Fußbett aufweist und der mit einer Decke versehen ist, welche zur individuellen Anpassung des Schuheinlegesohlen-Rohlings vom Grundkörper des Schuheinlegesohlen-Rohlings ablösbar ist, ohne dass dabei der Grundkörper oder die Decke beschädigt wird. Weiter soll die abgelöste Decke nach einer Anpassungsarbeit wieder auf den Grundkörper aufgebracht werden können und derart fest mit dem Grundkörper verbunden sein, dass die Decke bei normalen Einsatzbedingungen der Schuheinlage nicht verrutscht oder sich gar ablöst. Darüber hinaus soll das Ablösen als auch das erneute Aufbringen der Decke mit einfachen Mitteln möglich sein, ohne dass kostenintensive Hilfsmittel von Nöten sind.

Die Erfindung wird durch die unabhängigen Ansprüche definiert. Die abhängigen Ansprüche definieren bevorzugte Ausgestaltungen der Erfindung.

Die erfindungsgemäße Aufgabe wird mit einem Orthopädie-Schuheinlegesohlen-Rohling gemäß Anspruch 1 gelöst, der einen schuheinlegesohlen-artigen Grundkörper aus PU-Schaumkunststoff aufweist. Der Grundkörper des Schuheinlege-Rohlings weist auf der Oberseite ein dreidimensionales, orthopädisch wirksames Fußbett auf. Auf das PU-Schaumkunststoffmaterial des Grundkörpers ist allein mittels einer - entweder auf den Grundkörper oder auf einer Decke - aufgebrachten PU-Schicht eine Decke mit dem PU-Schaumkunststoff des Grundkörpers verbunden. Dabei kann der Grundkörper sowohl auf der Oberseite als auch auf der Unterseite eine mittels einer solchen PU-Schicht aufgebrachte Decke aufweisen. Der erfindungsgemäße Schuheinlegesohlen-Rohling weist hierzu eine spezielle PU-Schicht zwischen dem Grundkörper und der Ober- bzw. Unterdecke auf, die bereits bei Temperaturen über 40°C und unterhalb der Schmelztemperatur des PU-Schaumkunststoffes zum Ausbilden einer Haftverbindung aktivierbar ist, wobei die aktivierte PU-Schicht derart zähflüssig bleibt, dass sie nicht in das Material der jeweiligen Decke eindringt.

Erfindungsgemäß wird zum Aufbringen einer Decke auf die Ober- und/oder Unterseite des Grundkörpers aus PU-Material eine PU-Schicht als Haftmittel verwendet, wobei das für die PU-Schicht verwendete Polyurethan (PU) bei Temperaturen oberhalb von ca. 40°C, bevorzugt oberhalb von 50°C, aktivierbar ist, d. h. dessen Hafteigenschaften oberhalb der Aktivierungstemperatur stark ansteigen. Weiter soll die Aktivierungstemperatur unterhalb der Schmelztemperatur des PU-Materials liegen, aus dem der Grundkörper besteht. Damit kann die Decke nach Aktivieren der PU-Schicht auf den Grundköper aufgebracht werden, ohne dass es zu einem Verschweißen mit dem PU-Schaumkunststoff des Grundkörpers kommt. Nach einem Abkühlen der PU-Schicht haftet die Decke derart gut auf dem PU-Grundkörper, dass es bei normalen Einsatzbedingungen, insbesondere normalen Einsatztemperaturen, der Schuheinlage nicht zu einem Verrutschen oder Ablösen der Decke vom PU-Grundkörper kommt. Dabei wirkt sich die erfindungsgemäß gewählte Material-Paarung einer haftvermittelnden PU-Schicht zum Aufbringen einer Decke auf einen PU-Grundkörper günstig für die Adhäsion der Decke auf den Grundkörper aus.

Erfindungsgemäß soll eine PU-Masse als haftvermittelnde PU-Schicht verwendet werden, die oberhalb der Aktivierungstemperatur, aber unterhalb der Schmelztemperatur einen zählflüssigen, pastösen und gleichzeitig klebrigen Zustands annimmt, und zwar so dass das PU-Schichtmaterial auch im aktivierten Zustand nicht in das Material der Decke eindringt, insbesondere wenn das Deckenmaterial ein Textil oder ein textilähnliches Material ist.

Für die Verwirklichung der erfindungsgemäßen Idee ist es dabei unerheblich, ob die aktivierbare PU-Schicht vor dem Zusammenbringen des Grundkörpers und der Decke auf den Grundkörper oder auf die Rückseite der jeweiligen Decke aufgetragen wurde. In beiden Fällen kann die PU-Schicht beispielsweise über eine geeignete Wärmequelle, wie einen Heißluftfön, Infrarotlampen, Mikrowelle oder einfach durch einen Ofen auf Aktivierungstemperatur erwärmt werden. Dabei ist erfindungsgemäß die Schichtdicke der PU-Schicht im Vergleich zur Dicke der Decke bzw. des Grundkörpers sehr dünn, so dass ein Erwärmen der PU-Schicht schnell vonstattengeht. Nach der Erwärmung der PU-Schicht oberhalb der Aktivierungstemperatur kann der Grundkörper mit der Decke zusammengebracht werden, wobei dies sowohl mittels einer geeigneten Vorrichtung als auch händisch erfolgen kann. Dies ist insbesondere deshalb möglich, weil die PU-Schicht dünn ist, und so die zur Aktivierung benötigte Energie eine unnötige Aufheizung der Decke oder des Grundkörpers vermeidet, wodurch diese beiden Bauteile nur leicht erwärmt werden, und so während der Fügephase in der Hand gehalten werden können.

Je nach verwendeter PU-Masse kann die Aktivierung bei Temperaturen zwischen 40°C und etwa 100°C betragen. Auch wenn eine Aktivierungstemperatur unterhalb 50°C möglich erscheint und vom Erfindungsgedanken umfasst ist, so sollte diese bevorzugt nicht unterschritten werden, um den festen Halt der Decke auf den PU-Schaumkunststoffgrundkörper nicht zu verringern. Die Körpertemperatur des Menschen liegt bei etwa 37°C, so dass es in heißen Sommertagen beim Gehen auf heißem Asphalt durchaus zu Temperaturen über 40°C im Schuh kommen kann, wodurch gegebenenfalls eine Relativbewegung zwischen der Decke und Schuheinlegesohlengrundkörper auftreten könnte. Es ist daher bevorzugt, dass die Aktivierungstemperatur der verwendeten PU-Masse oberhalb von 50°C liegt, damit solche Relativbewegungen, d. h. ein Verrutschen der Decke ausgeschlossen bleiben.

Die Wahl der Aktivierungstemperatur unterhalb der Schmelztemperatur des PU-Schaumkunststoffs des Grundkörpers vermeidet einerseits, dass der Energieeintrag beim Verkleben der Decke mit dem Grundkörper nicht zu hoch wird und andererseits die verwendeten Materialen für die Decke als auch für den Grundkörper nicht angeschmolzen werden, was ein späteres zerstörungsfreies Ablösen verhindern würde. Dies ist darüber hinaus weiter bevorzugt, damit die Decke nach einem Erwärmen bzw. Aktivieren per Hand auf dem Grundkörper während der Abkühlphase positionierbar und auch manuell festhaltbar ist.

Die Verwendung einer zähflüssigen PU-Masse mit einer Aktivierungstemperatur unterhalb von etwa 100°C ermöglicht weiterhin ein erneutes Ablösen der Decke vom Grundkörper des Schuheinlegesohlen-Rohlings. Nachdem der Schuheinlegesohlen-Rohling mit der darauf aufgebrachten Decke auf eine Temperatur erwärmt wird, die unterhalb der Schmelztemperatur der verwendeten Materialen für die Decke oder des PU-Grundkörpers, d. h. des PU-Schaumkunststoffs liegt, jedoch oberhalb der Aktivierungstemperatur des PU-Schichtmaterials, kann die Decke zerstörungsfrei vom Grundkörper abgelöst werden, wobei weder der Grundkörper noch die Decke beschädigt wird.

Mit der erfindungsgemäßen Verwendung einer zähflüssigen PU-Schicht, welche bei Temperaturen oberhalb etwa 40°C jedoch unterhalb von etwa 100°C aktivierbar ist, wird somit eine unter Normalbedingungen zuverlässige Verbindung der Decke mit dem Schuheinlegesohlen-Grundkörper erreicht, die mit einfachen Mitteln, insbesondere per Hand, bewerkstelligt werden kann. Hierbei ist insbesondere das Verkleben als auch das Ablösen und das erneute Verkleben der Decke mit dem PU-Grundkörper zu nennen. Speziell die Möglichkeit des erneuten Aufbringens der Decke auf den Grundkörper nach einem vorangegangenen Ablösen bietet vielfältige Möglichkeiten bei der individuellen Anpassung des Schuheinlegesohlen-Rohlings an die individuelle Fußsohlenform des Trägers der Schuheinlegesohle.

Bei dem erfindungsgemäß verwendeten Material für die bevorzugt dünne und thermisch aktivierbare PU-Schicht handelt es um ein aktivierbares PU-Material, das PU-Gussmaterial, einen Kleber auf PU-Basis, thermoplastisches Polyurethan (TPU) oder eine sonstige chemische Zusammensetzung aufweist, die unterhalb von 40°C ein gutes Adhäsionsverhalten mit dem Schaumkunststoff des Grundkörpers zeigt. Jedoch erweicht das Material der erfindungsgemäß verwendeten PU-Schicht oberhalb der Aktivierungstemperatureine derart, dass es sich auf den PU-Schaumkunststoff des Grundköpers ggf. unter plastischer Verformung der PU-Schicht auftragen lässt und, ggf. nach erneutem thermischen Aktivieren, von dem PU-Grundköper wieder abziehen lässt, ohne dass dabei der Grundköper oder die Decke zu Schaden kommt. Mit anderen Worten, oberhalb der Aktivierungstemperatur lässt sich die Decke in Art eines Haftetiketts auf den Grundkörper aufbringen und von diesem wieder lösen.

So kann ein Schuheinlegesohlen-Rohling mit einer Grundform für ein orthopädisches Fußbett mit aufgebrachter Decke vom Hersteller des Rohlings "vormontiert" an Schuhorthopäden bzw. Orthopädietechniker ausgeliefert werden, ohne dass hierbei zwei separate Bauteile, wie bisher im Stand der Technik üblich, versendet werden müssen. Ferner kann der meist aufwendige Zuschnitt der Decke beim Orthopäden bzw. Orthopädietechniker entfallen, da dieser Zuschnitt bereits beim Hersteller des Schuheinlege-Rohlings erfolgen kann. Dem Orthopädietechniker bietet sich trotzdem die Möglichkeit den Schuheinlegesohlen-Grundkörper auf allen Oberflächen bearbeiten zu können, indem er die Decke nach Erwärmen des Rohlings zumindest teilweise oder komplett entfernen kann und durch Aktivieren der zähflüssigen PU-Schicht die Decke wieder mit dem Grundkörper verbinden kann. So ist durch geringen Aufwand die Grundform, welche vom Hersteller der Schuheinlegesohlen-Rohlinge zur Verfügung gestellt wird, individuell an die Fußsohle eines Trägers einer Schuheinlegesohle bzw. auch an den Schuh, in den die Schuheinlegesohle hineingelegt werden soll, einfach anpassbar, wobei Komfort oder funktionale Decken bereits in geeigneter Form und Größe als Sohlenkomponente zur Verfügung stehen.

Dies spart insbesondere beim Schuhorthopädietechniker einen erhöhten Arbeitsaufwand für die Vervollständigung einer Schuheinlegesohle, um die individuellen Bedürfnisse und Wünsche des Trägers der Schuheinlegesohle zu erfüllen.

In einer bevorzugten Ausführungsform wird die erfindungsgemäße Verwendung einer zähflüssigen PU-Masse zum Verkleben einer Decke mit dem Grundkörper eines Schuheinlege-Rohlings bevorzugt auf die Rückseite der Decke aufgetragen, also die Seite, die mit dem Grundkörper des Schuheinlegesohlen-Rohlings in Kontakt kommt. Dabei ist weiter bevorzugt, dass die Verbindung zwischen der PU-Schicht und der Decke stabiler ist als die Verbindung zwischen PU-Schicht und PU-Grundkörper, damit nach einem Ablösen der Decke vom Grundkörper die PU-Schicht im Wesentlichen auf der Decke verbleibt. Somit kann der Grundkörper nach Ablösen der Decke auch auf der zuvor verdeckten Oberfläche beispielsweise durch Fräsen, Schleifen, Warmverformen, einem plastischen Verformen oder Zuschneiden und/oder auch durch Aufbringen einer Pelotte orthopädisch an die Fußsohle des Trägers der Schuheinlegesohle angepasst werden. Anschließend kann dieselbe Decke wieder auf den Grundkörper aufgebracht werden, indem lediglich die PU-Schicht auf Aktivierungstemperatur erwärmt wird.

In einer weiteren bevorzugten Ausführungsform ist der Grundkörper mittels RIM-Verfahren in einer Schäumform als Fräsblock oder als bereits vorkonturierter Schuheinlegesohlen-Rohling ausgebildet. Vom Erfindungsgedanken ist hierbei auch eine Teilform einer Schuheinlegesohle, insbesondere eine Halbsohle umfasst, wobei gegebenenfalls die Decke die Schuhsohlenform komplettiert. Selbstredend ist hierbei bevorzugt, dass die haltvermittelnde PU-Schicht nur in dem Bereich aufgetragen ist, in dem der Grundkörper und die Decke einander überlappen. Weitere Beispiele hierfür sind Gewölbe- oder Fersenkeile oder ähnliche in der Orthopädie verwendete Elemente, die eine orthopädische Wirkung auf den Träger der Schuheinlegesohle haben.

Insgesamt wird einem Orthopäden bzw. einem Orthopädietechniker eine einfache Möglichkeit zur Bearbeitung einer Grundform für einen Schuheinlegesohlen-Rohling zur Verfügung gestellt, bei dem er den bereits mit einer Decke versehenen Schuheinlegesohlen-Grundkörper aus PU-Material auf allen Seiten bearbeiten und so die Grundform individuell an die Fußsohle des Trägers der Schuheinlegsohle anpassen kann. Auch die Anpassung des erfindungsgemäßen Schuheinlegesohlen-Rohlings an die Schuhinnenform, in die die Schuheinlegesohle eingelegt werden soll, ist vom Erfindungsgedanken umfasst. Weiter bevorzugt wird bei der Anpassung an beide Seiten ein optimales orthopädisches Ergebnis erreicht. Dabei wird dem Schuhorthopäden der Aufwand des Zuschneidens und Auftragens von Kleber oder ähnlichen Hilfsmitteln zum Verbinden der Decke mit dem Schuheinlegesohlen-Grundkörper abgenommen, was nicht nur eine Arbeitserleichterung darstellt, sondern auch das umständliche und oft unsaubere Handling von Klebstoffen vermeidet, mit dem damit verbundenen Verschmutzen der Schuheinlage und der Werkzeuge bzw. Vorrichtungen beim Orthopäden.

Der Schuhorthopäde bzw. Schuhorthopädietechniker kann, nach Erwärmen des erfindungsgemäßen Schuheinlegesohlen-Rohlings mit aufgebrachter Decke auf eine Temperatur unterhalb der Schmelztemperatur des PU-Schaumkunststoffes des Grundkörpers, die Decke vom Grundkörper ablösen, den Grundkörper an die Fußsohlenform des Trägers bzw. an die Schuhinnenform in den die Schuheinlegesohle eingelegt werden soll, anpassen und nach erneutem Erwärmen/Aktivieren der PU-Schicht auf eine Temperatur oberhalb von 40°C, jedoch unterhalb von der Schmelztemperatur des PU-Grundkörpermaterials, die Decke mittels Druck erneut auf den Grundkörper aufbringen. Hier kann der Orthopäde bzw. Schuhorthopädietechniker dieselbe Decke, die er zuvor vom Grundkörper der Schuheinlegsohle gelöst hat, wieder auf den bearbeiteten Grundkörper aufbringen, ohne dass er diese erneut mit einem Klebstoff, wie beispielsweise Heißkleber oder ähnlichem, versehen muss. Er muss lediglich dafür sorgen, dass die PU-Schicht die Aktivierungstemperatur erreicht, wodurch er die Decke beispielsweise manuell auf den Grundkörper drücken kann. Nach Unterschreiten der Aktivierungstemperatur erhält er einen festen Verbund der Decke mit dem Grundkörper als Schuheinlage, die individuell an die Fußform angepasst ist. Dabei ist der Orthopäde bzw. Orthopädiefachmann in der Wahl seiner Mittel sowohl für das Ablösen als auch für das erneute Aufbringen der Decke frei. Beispielsweise ist ein einfacher Ofens ausreichend, in dem er die Sohle mit der aufgebrachten Decke erwärmen kann, oder in dem er auch nur die Decke erwärmt, für das erfindungsgemäße Ablösen und erneute Aufbringen der Decke. Andere Wärmequellen stehen dem Fachmann dabei selbstredend ebenfalls zur Verfügung, wie beispielsweise ein Mikrowellenofen, ein Heißluftfön, eine einfache erwärmte Platte, etc. Insbesondere ein Fachmann wird hierfür vielfältige Lösungen finden, die allesamt vom Erfindungsgedanken umfasst sind.

Auch eine automatisierte Aufbringung der Decke, insbesondere beim Hersteller des Schuheinlegesohlen-Rohlings ist im Bereich des Könnens des einschlägigen Fachmanns und ist mit verschiedenen Möglichkeiten, wie z. B. einer Abwandlung einer Bügelvorrichtung oder mittels einer Infrarot- oder Mikrowellenstrecke und nachgeschalteter Presse vorstellbar.

Selbstredend ist die Auslieferung des erfindungsgemäßen Schuheinlagesohlen-Rohlings mit nicht-aufgebrachter Decke vom Erfindungsgedanken umfasst, wobei die Decke oder Grundkörper mit einer PU-Schicht versehen ist, die oberhalb einer Temperatur von 40°C, bevorzugt oberhalb 50°C, für das Verbinden mit dem Schuheinlegesohle-Grundkörper vorgesehen ist. Bei einer solchen separaten Auslieferung von Decke und Grundkörper ist es bevorzugt, die auf einen der beiden Teile aufgebrachte PU-Masse mit einer Schutzfolie zu versehen. Konsequenterweise umfasst der Erfindungsgedanke auch die eine Auslieferung des Schuheinlegesohlen-Grundkörpers mit einer aufgebrachten erfindungsgemäß aktivierbaren PU-Schicht, jedoch ohne Decke, da eine solche beispielsweise beim Orthopäden zum Vervollständigen der erfindungsgemäßen Schuheinlegesohle hinzugefügt werden kann.

Im Folgenden werden zur Veranschaulichung des Erfindungsgedankens schematisch einige Ausführungsbeispiele eines erfindungsgemäßen Schuheinlegesohlen-Rohlings mit einem dreidimensional ausgebildeten, orthopädischen Fußbett dargestellt, wobei diese Ausführungsformen rein der Erläuterung des Erfindungsgedankens dienen und diesen nicht einschränken. Es zeigen:
- Figur 1: einen Schuheinlegesohlen-Rohling gemäß der Erfindung in einer ersten Ausführungsform
- Figur 2: einen erfindungsgemäßen Schuheinlegesohlen-Rohling gemäß einer zweiten Ausführungsform
- Figur 3: einen erfindungsgemäßen Schuheinlegesohlen-Rohling gemäß einer zweiten Ausführungsform
- Figur 4: einen Querschnitt durch den erfindungsgemäßen Schuheinlegesohlen-Rohling gemäß Figur 3 längs der Linie A-A
- Figur 5: einen Längsschnitt durch den erfindungsgemäßen Schuheinlegesohlen-Rohling gemäß Figur 3 längs der Linie B-B;

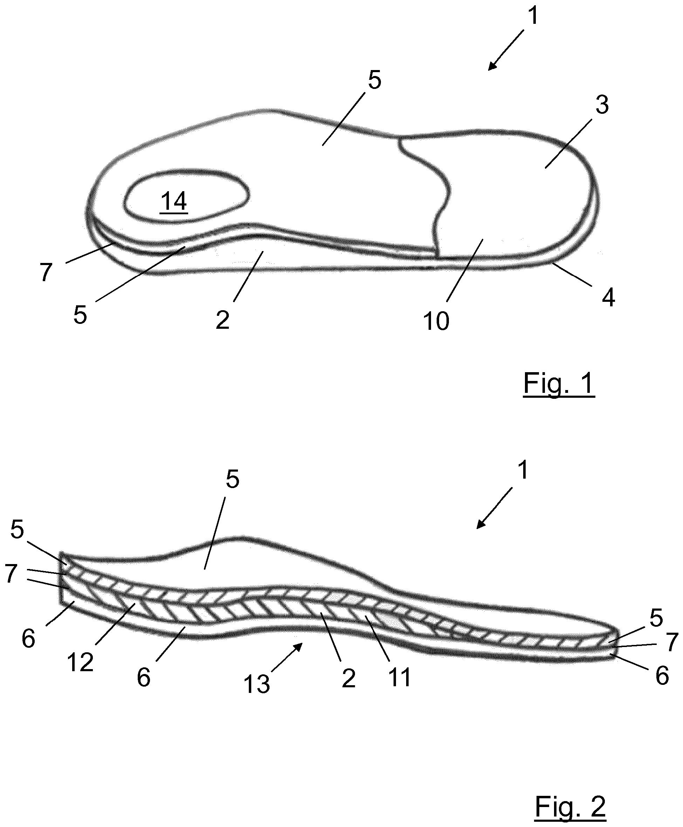

Figur 1 zeigt schematisch eine perspektivische Ansicht eines ersten Ausführungsbeispiels gemäß der Erfindung. Der in Figur 1 gezeigte Schaumkunststoff-Schuheinlegesohlen-Rohling 1 weist auf seiner Oberseite 3 ein dreidimensional ausgebildetes orthopädisch wirksames Fußbett auf. Dabei ist der Grundkörper 2 aus PU-Schaumkunststoffmaterial ausgebildet und wurde bevorzugt im RIM-Schäumverfahren erstellt. Auf den Grundkörper 2 nur ist in Teilbereichen eine Oberdecke 5 aufgebracht, sodass beispielsweise der Vorderfußbereich 10 und der Bereich der Fersenmulde 14 nicht bedeckt sind. Die Oberdecke 5 wurde mittels einer PU-Schicht 7 auf das PU-Schaumkunststoffmaterial des Grundkörpers 2 aufgebracht. Dabei ist die zum Verbinden der Oberdecke 5 mit der Oberseite 3 des Grundkörpers 2 vorgesehene PU-Schicht im Verhältnis zu den Schichtdicken der Oberdecke 5 und des Grundkörpers 2 derart dünn, dass sie in Figur 1 als Linie erscheint. Die PU-Schicht, wie oben ausführlich erläutert, ist dabei eine mittels Wärme aktivierbare PU-Masse, welche bei Temperaturen bevorzugt oberhalb von 40° C einen deutlichen Anstieg der Adhäsionseigenschaften zeigt, jedoch derart zähflüssig ist/bleibt, dass das Material der PU-Schicht nicht in das Material Oberdecke 5 eindringt. Wie oben ebenfalls bereits ausgeführt, liegt die Adhäsionsaktivierungstemperatur des Materials der PU-Schicht unterhalb der Schmelztemperatur des PU-Schaumkunststoffmaterials des Grundkörpers 2. Damit wird eine Adhäsion zwischen Oberdecke 5 und Grundkörper 2 erreicht, ohne dass die Schmelztemperatur eines der beiden Adhäsionspartner überschritten wird. Weiter findet dadurch beim Verbinden bzw. Aufbringen der Oberdecke 5 auf den Grundkörper 2 kein Verschweißen der PU-Materialien der PU-Schicht und dem PU-Schaumkunststoff des Grundköpers 2 statt.

Figur 2 zeigt einen Längsschnitt durch ein zweites Ausführungsbeispiel der Erfindung. Im Unterschied zum Ausführungsbeispiel der Figur 1 ist der Grundkörper im Ausführungsbeispiel der Figur 2 in Art einer Halbsohle ausgebildet, d. h. er erstreckt sich lediglich vom Fersenbereich 12 bis zum Mittelfußbereich 11, wobei der Vorderfußbereich 10 vom Grundkörper 2 nicht unterstützt wird. In Figur 2 ist jedoch deutlich erkennbar, dass durch den Grundkörper 2 im Mittelfußbereich ein Fußstützendes Gewölbe ausgebildet wird, sodass der Grundkörper 2 zumindest hierdurch eine orthopädische Funktion aufweist. Als weiteren Unterschied zeigt der Schuheinlegesohlen-Rohling gemäß Figur 2 eine Oberdecke 5 als auch eine Unterdecke 6, welche jeweils mittels einer PU-Schicht 7 mit der jeweiligen Oberseite 3 bzw. der Unterseite 4 mit dem im Fersen- und Mittelbereich ausgebildeten Grundkörper 2 verbunden sind. Im Vorderfußbereich 10 sind die beiden zueinander weisenden Seiten der Oberdecke 5 und der Unterdecke 6 bspw. direkt über eine PU-Schicht 7 miteinander verbunden, die zuvor entweder auf die Oberdecke 5 oder die Unterdecke 6 aufgetragen wurde. Wie oben bereits ausgeführt, reicht ein einseitiger Auftrag der PU-Schicht 7 für eine erfindungsgemäße mittels Wärme wieder lösbare Verbindung zweier PU-Bauteile. Das bedeutet weiter, dass in diesem Ausführungsbeispiel - falls eine der beiden Decken 5 oder 6 nicht aus PU-Material besteht - diejenige Decke 5 oder 6 vorzugsweise ebenfalls eine PU-Schicht aufweist, die dann nicht zwingend eine mittels Wärme aktivierbare haftvermittelnde PU-Schicht gemäß der Erfindung sein muss.

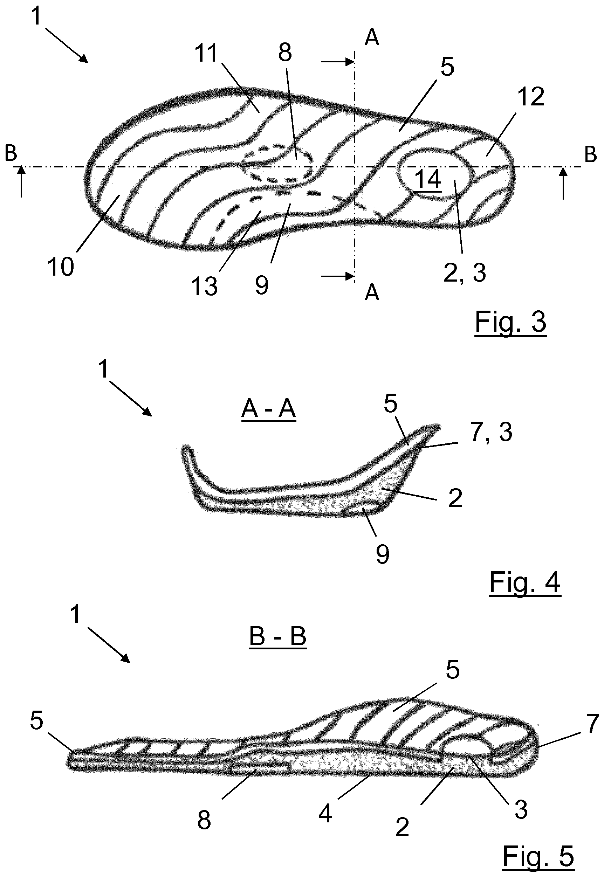

Figur 3 zeigt eine Draufsicht eines weiteren Ausführungsbeispiels der Erfindung. Der in Figur 3 dargestellte orthopädische Schuheinlegesohlen-Rohling 1 ist auf der Oberseite 3 vollflächig mit einer Oberdecke 5 bezogen, mit Ausnahme der Fersenmulde 14 im Fersenbereich 12. Mit gestrichelten Linien ist in Figur 3 angedeutet, dass der erfindungsgemäße Schuheinlegesohlen-Rohling 1 im Mittelfußbereich 11 sowohl eine Pelotte 8 als auch einen Gewölbekeil 9 aufweist. Den Gewölbekeil 9 erkennt der Fachmann im Schnitt A-A quer durch den Schuheinlegesohlen-Rohling 1, der in Figur 4 dargestellt ist. Aus dieser Darstellung erkennt der Fachmann ebenfalls, dass die Oberdecke 5 mittels einer PU-Schicht 7 auf die Oberseite 3 des Grundkörpers 2 aufgebracht ist.

Der in Figur 5 dargestellte Längsschnitt des Schuheinlegesohlen-Rohlings 1 gemäß Figur 3 verdeutlicht den vollflächigen Bezug der Oberseite 3 des Grundkörpers 2 mit einer Oberdecke 5, welche haftvermittelnd mittels einer PU-Schicht 7 auf den Grundkörper aufgezogen ist. In Figur 5 erkennt man weiterhin die im Mittelfußbereich 11 angeordnete Pelotte 8 im Schnitt.

Auch wenn der in den Figuren 3 bis 5 dargestellte Schuheinlegesohlen-Rohling 1 keine Unterdecke aufweist, so erkennt zumindest der einschlägige Fachmann, dass eine Unterdecke - wie beispielsweise in Figur 2 gezeigt - ebenfalls auf den Schuheinlegesohlen-Rohling 1 gemäß den Figuren 3 bis 5 mit einer erfindungsgemäßen PU-Schicht aufgezogen werden kann. Der Fachmann erkennt weiter, dass auch - beispielsweise wie in Figur 1 angedeutet - teilflächige Decken auf bereits vorhandene Decken aufgezogen werden können. Dies ist beispielsweise mit dem in Figur 2 gezeigten Längsschnitt im Vorderfußbereich 10 verwirklicht, da man dort die Unterdecke beispielsweise nur teilflächig ausbilden könnte oder Durchbrüche/Öffnungen in der Oberdecke im Vorderfußbereich belässt, sodass durch diese Öffnungen in der Oberdecke die Unterdecke sichtbar ist. Allgemein sind dem Fachmann keine Grenzen gesetzt, wie er Kombinationen zum Beziehen des PU-Schaumkunststoffgrundkörpers 2 auf dessen Oberseite 3 oder Unterseite 4 gestalten kann, wobei Kombinationen von ein- oder mehrlagigen Oberdecken 5 und Unterdecken 6 genauso im Bereich des Könnens des einschlägigen Fachmann sind.

Insgesamt wird mit der erfindungsgemäßen Verwendung einer wärmeaktivierbaren PU-Schicht zum Erreichen einer Adhäsion zwischen einer Schuhsohlendecke und einem Schuhsohlengrundkörper eine, insbesondere für die Orthopädie einfache, kostengünstige, jedoch flexible und saubere Lösung angeboten, einen Schuheinlegesohlen-Rohling individuell an die Fußsohle des Trägers der Schuheinlegesohle bzw. an die Schuhinnenform, in den die Schuheinlegesohle eingelegt werden soll, orthopädisch anzupassen. Dabei erhält der Fachmann viele Möglichkeiten, die Schuheinlegesohle orthopädisch, wie gestalterisch mit einfachen Mitteln anzupassen, da er entweder zum Aufbringen der Decke lediglich die PU-Schicht über Aktivierungstemperatur erwärmen muss, oder - wenn er eine bereits aufgebrachte Decke vom Grundkörper oder von einer anderen Decke lösen will - nur dafür Sorge tragen muss, dass die Schuheinlegesohlen-Teile soweit erwärmt werden, dass die Aktivierungstemperatur in der PU-Schicht erreicht wird. Hierzu reichen ihm einfache Möglichkeiten, wie ein Ofen, ein Heißluftföhn oder eine Wärmeplatte, etc.

### Bezugszeichenliste

- 1: Schuheinlagesohlen-Rohling
- 2: Grundkörper
- 3: Oberseite
- 4: Unterseite
- 5: Oberdecke
- 6: Unterdecke
- 7: PU-Schicht
- 8: Pelotte
- 9: Gewölbekeil
- 10: Vorderfußbereich
- 11: Mittelfußbereich
- 12: Fersenbereich
- 13: Gewölbereich
- 14: Fersenmulde

## Patentansprüche

1. Orthopädischer Schuheinlegesohlen-Rohling (1), der aufweist:
• einen Schuheinlegesohlen-artigen Grundköper (2) aus PU-Schaumkunststoff,
• ein auf der Oberseite (3) des Grundköpers (2) ausgebildetes, dreidimensionales Fußbett, und
• eine auf der Ober- und/oder Unterseite (3, 4) aufgebrachte Decke (5, 6), die allein mittels einer PU-Schicht mit dem PU-Schaumkunststoff des Grundkörpers verbunden ist,
**dadurch gekennzeichnet, dass**
• die PU- Schicht ein zum Ausbilden einer Haftverbindung der Decke (5, 6) mit dem Grundkörper bei Temperaturen über 40°C und unterhalb der Schmelztemperatur des PU-Schaumkunststoffs aktivierbares PU-Material ist, das im aktivierten Zustand derart zähflüssig ist, dass es nicht in das Material der Decke (5, 6) eindringt, und
• die Decke (5, 6) jederzeit nach erneutem Erwärmen der PU- Schicht auf eine Temperatur über 40°C jedoch unterhalb der Schmelztemperatur des PU-Schaumkunststoffs vom Grundkörper abgelöst werden kann, wobei weder der Grundkörper noch die Decke (5, 6) beschädigt wird.

2. Schuheinlegesohlen-Rohling (1) nach Anspruch 1, wobei der Grundköper (2) mittels RIM-Verfahren in einer oder mehreren Materialhärten hergestellt wurde.

3. Schuheinlegesohlen-Rohling (1) nach einem der Ansprüche 1 oder 2, wobei der Grundkörper (2) vor einem Aufbringen der Decke (5, 6) durch Fräsen und/oder Schleifen bearbeitet wurde.

4. Schuheinlegesohlen-Rohling (1) nach einem der Ansprüche 1 bis 3, wobei der Grundköper (2) nach einem Ablösen der Decke (5, 6) an die Fußsohlen-Form eines Trägers der Schuheinlegesohle anpassbar ist und die zuvor abgelöste Decke (5, 6) nach dem Anpassen des Grundköpers durch Erwärmen der PU-Schicht auf über 40°C und unterhalb der Schmelztemperatur des PU-Schaumkunststoffs wieder auf den Grundköper (2) aufgebracht werden kann.

5. Schuheinlegesohlen-Rohling (1) nach einem der Ansprüche 1 bis 4, wobei die Decke (5, 6) mit der auf über 40°C und unterhalb der Schmelztemperatur des PU-Schaumkunststoffs erwärmten PU-Schicht von Hand auf den Grundkörper aufgebracht und festgedrückt werden kann.

6. Schuheinlegesohlen-Rohling (1) nach einem der Ansprüche 1 bis 5, wobei das Deckenmaterial Leder, Alcantara, ein gewebtes oder gewirktes Textil, ein Vlies, Fell oder ein anderes fachübliches Deckenmaterial oder eine Schutzschicht bzw. Schutzfolie ist.

7. Schuheinlegesohlen-Rohling (1) nach einem der Ansprüche 1 bis 6, wobei das Material der PU-Schicht ein Thermoplastisches Polyurethan (TPU) ist.

8. Verfahren zum individuellen Anpassen eines mit einer Decke (5, 6) versehenen Schuheinlegesohlen-Rohlings (1) nach einem der Ansprüche 1 bis 7 an die Fußsohlen-Form eines Trägers der Schuheinlegesohle und/oder Schuhinnenform in die die Schuheinlegesohle eingelegt werden soll, mit den folgenden Schritten:
a) Erwärmen der den Grundkörper (2) und die Decke (5, 6) verbindenden PU-Schicht auf eine Temperatur über 40°C jedoch unterhalb der Schmelztemperatur des PU-Schaumkunststoff des Grundköpers (2);
b) Ablösen der Decke (5, 6) vom Grundkörper (2);
c) Anpassen des Grundköpers an die Fußsohlen-Form und /oder Schuhinnenform;
d) Erwärmen der PU-Schicht auf der Decke (5, 6) oder auf dem Grundkörper (2) zumindest auf der Seite, die mit dem Grundköper (2) bzw. der Decke (5, 6) in Kontakt kommen soll, auf eine Temperatur oberhalb von 40°C, jedoch unterhalb der Schmelztemperatur des PU-Schaumkunststoffs des Grundköpers,
e) Aufbringen mittels Druck der im Schritt d) erwärmten Decke (5, 6) auf den Grundköper (2);

9. Verfahren nach Anspruch 8, bei dem das Erwärmen in den Schritten a) und/oder d) mittels eines Ofen, einer erwärmten Platte oder Walze, in einem Heißluftstrom oder mittels Mikrowellen erfolgt.

10. Verfahren nach Anspruch 8 oder 9, bei dem das Ablösen im Schritt b) manuell erfolgt.

11. Verfahren nach einem der Ansprüche 8 bis 10, bei dem das Aufbringen im Schritt e) von Hand erfolgt.

12. Verfahren nach einem der Ansprüche 8 bis 11, bei dem die Decke (5, 6) nach dem Schritt c) mit der PU-Schicht zum Grundköper (2) weisend auf den Grundköper (2) gelegt wird und die Schritte d) und e) durch eine Bügelvorrichtung vorgenommen werden.

13. Verfahren nach einem der Ansprüche 8 bis 12, bei dem das Anpassen des Grundköpers an die Fußsohlen-Form des Trägers durch Fräsen, Schleifen, Warmverformen, plastisch Verformen, Zuschneiden des Grundköpers und/oder durch Aufbringen einer Pelotte auf den Grundköper (2) erfolgt.

## Claims

1. Orthopaedic shoe insole blank (1) having:
• a main body (2) shaped like a shoe insole and made of PU foam,
• a three-dimensional footbed formed on the upper side (3) of the main body (2), and
• a cover (5, 6), which is applied to the upper side and/or underside (3, 4) and is connected to the PU foam of the main body by means of a PU layer alone,
**characterized in that**
• the PU layer is a PU material which can be activated to adhesively connect the cover (5, 6) to the main body at temperatures above 40°C and below the melting point of the PU foam and which, in the activated state, is of such a viscosity that it does not penetrate into the material of the cover (5, 6), and,
• after renewed heating of the PU layer to a temperature above 40°C but below the melting point of the PU foam, the cover (5, 6) can be detached at any time from the main body, with neither the main body nor the cover (5, 6) being damaged.

2. Shoe insole blank (1) according to Claim 1, wherein the main body (2) was produced in one or more material hardnesses by means of RIM methods.

3. Shoe insole blank (1) according to either of Claims 1 and 2, wherein the main body (2) was processed by milling and/or grinding before an application of the cover (5, 6).

4. Shoe insole blank (1) according to one of Claims 1 to 3, wherein the main body (2), after a detachment of the cover (5, 6), can be adapted to the shape of the sole of a wearer of the shoe insole, and, after the adaptation of the main body, the cover (5, 6) detached beforehand can be applied again to the main body (2) by heating the PU layer to above 40°C and below the melting point of the PU foam.

5. Shoe insole blank (1) according to one of Claims 1 to 4, wherein the cover (5, 6), with the PU layer heated to above 40°C and below the melting point of the PU foam, can be applied to the main body by hand and firmly pressed.

6. Shoe insole blank (1) according to one of Claims 1 to 5, wherein the cover material is leather, Alcantara, a woven or knitted textile, a nonwoven, fur or another customary cover material or a protective layer or a protective film.

7. Shoe insole blank (1) according to one of Claims 1 to 6, wherein the material of the PU layer is a thermoplastic polyurethane (TPU).

8. Method by which a shoe insole blank (1) according to one of Claims 1 to 7, provided with a cover (5, 6), is individually adapted to the shape of the sole of a wearer of the shoe insole and/or to the inner shape of the shoe into which the shoe insole is intended to be placed, said method having the following steps:
a) the PU layer connecting the main body (2) and the cover (5, 6) is heated to a temperature above 40°C but below the melting point of the PU foam of the main body (2);
b) the cover (5, 6) is detached from the main body (2);
c) the main body is adapted to the shape of the sole and/or the inner shape of the shoe;
d) the PU layer on the cover (5, 6) or on the main body (2) is heated, at least on the side intended to come into contact with the main body (2) or the cover (5, 6), to temperature above 40°C but below the melting point of the PU foam of the main body,
e) the cover (5, 6) heated in step d) is applied to the main body (2) by means of pressure.

9. Method according to Claim 8, in which the heating in steps a) and/or d) is carried out by means of an oven, a heated plate or roller, in a hot-air stream or by means of microwaves.

10. Method according to Claim 8 or 9, in which the detachment in step b) is carried out manually.

11. Method according to one of Claims 8 to 10, in which the applying in step e) is carried out by hand.

12. Method according to one of Claims 8 to 11, in which the cover (5, 6), after step c), is placed onto the main body (2), with the PU layer facing the main body (2), and steps d) and e) are carried out by an ironing device.

13. Method according to one of Claims 8 to 12, in which the adaptation of the main body to the shape of the sole of the wearer is carried out by milling, grinding, hot forming, plastic deformation, cutting of the main body to size, and/or by applying a pad to the main body (2).

## Revendications

1. Ébauche de semelle intérieure orthopédique (1), comportant :
• un corps de base (2) de type semelle intérieure en mousse de plastique PU,
• une assise de pied tridimensionnelle formée sur la face supérieure (3) du corps de base (2), et
• une couverture (5, 6) appliquée sur la face supérieure et/ou inférieure (3, 4), qui est uniquement reliée au moyen d'une couche de PU à la couche de plastique PU du corps de base,
**caractérisée** en ce
• la couche de PU est un matériau de PU activable destiné à créer une liaison d'adhérence de la couverture (5, 6) avec le corps de base à des températures supérieures à 40 °C et inférieures à la température de fusion de la couche de plastique PU, qui est, à l'état activé, si visqueux qu'il ne pénètre pas dans le matériau de la couverture (5, 6), et
• la couverture (5, 6) peut, à tout moment suivant un nouveau réchauffement de la couche de PU à une température supérieure à 40 °C mais inférieure à la température de fusion de la couche de plastique PU, être détachée du corps de base, le corps de base et la couverture (5, 6) n'étant pas endommagés.

2. Ébauche de semelle intérieure (1) selon la revendication 1, le corps de base (2) étant fabriqué au moyen d'un procédé RIM dans une ou plusieurs duretés de matériau.

3. Ébauche de semelle intérieure (1) selon l'une quelconque des revendications 1 ou 2, le corps de base (2) ayant été traité par fraisage et/ou ponçage avant l'application de la couverture (5, 6).

4. Ébauche de semelle intérieure (1) selon l'une quelconque des revendications 1 à 3, le corps de base (2) pouvant, après détachement de la couverture (5, 6), être adapté à la forme de semelle d'un porteur de la semelle intérieure et la couverture (5, 6) préalablement détachée après l'adaptation du corps de base par réchauffement de la couche de PU à une température supérieure à 40 °C et inférieure à la température de fusion de la couche de plastique PU pouvant être à nouveau appliquée sur le corps de base (2).

5. Ébauche de semelle intérieure (1) selon l'une quelconque des revendications 1 à 4, la couverture (5, 6) pourvue de la couche de PU réchauffée à une température supérieure à 40 °C et inférieure à la température de fusion de la couche de plastique PU pouvant être manuellement appliquée et pressée fermement sur le corps de base.

6. Ébauche de semelle intérieure (1) selon l'une quelconque des revendications 1 à 5, le matériau de couverture étant du cuir, de l'Alcantara, un textile tissé ou tricoté, un non-tissé, une fourrure ou tout autre matériau de couverture usuel dans la profession, une couche de protection ou un film de protection.

7. Ébauche de semelle intérieure (1) selon l'une quelconque des revendications 1 à 6, le matériau de la couche de PU étant un polyuréthane thermoplastique (TPU).

8. Procédé destiné à l'adaptation individuelle d'une ébauche de semelle intérieure (1) pourvue d'une couverture (5, 6) selon l'une quelconque des revendications 1 à 7 à la forme de semelle du porteur d'une semelle intérieure et/ou à la forme intérieure de la chaussure dans laquelle la semelle intérieure doit être insérée, comprenant les étapes suivantes :
a) réchauffement de la couche de PU reliant le corps de base (2) et la couverture (5, 6) à une température supérieure à 40 °C mais inférieure à la température de fusion de la couche de plastique PU du corps de base (2) ;
b) détachement de la couverture (5, 6) du corps de base (2) ;
c) adaptation du corps de base à la forme de la semelle et /ou à la forme intérieure de la chaussure ;
d) réchauffement de la couche de PU sur la couverture (5, 6) ou sur le corps de base (2) au moins du côté devant entrer en contact avec le corps de base (2) ou la couverture (5, 6) à une température supérieure à 40 °C mais inférieure à la température de fusion de la mousse de plastique PU du corps de base ;
e) application par pression de la couverture (5, 6) réchauffée à l'étape d) sur le corps de base (2) ;

9. Procédé selon la revendication 8, dans lequel le réchauffement au cours des étapes a) et/ou d) est réalisé au moyen d'un four, d'une plaque ou d'un rouleau réchauffé, dans un courant d'air chaud ou au moyen de micro-ondes.

10. Procédé selon la revendication 8 ou 9, dans lequel le détachement à l'étape b) est réalisé manuellement.

11. Procédé selon l'une quelconque des revendications 8 à 10, dans lequel l'application à l'étape e) est réalisée manuellement.

12. Procédé selon l'une quelconque des revendications 8 à 11, dans lequel la couverture (5, 6) est posée, après l'étape c), sur le corps de base (2) avec la couche de PU orientée vers le corps de base (2), et les étapes d) et e) sont réalisées avec un dispositif de repassage.

13. Procédé selon l'une quelconque des revendications 8 à 12, dans lequel l'adaptation du corps de base à la forme de la semelle du porteur est réalisée par fraisage, ponçage, thermoformage, formage plastique, découpe du corps de base et/ou la pose d'une pelote sur le corps de base (2).
